# EUROPEAN PATENT APPLICATION

(11) **EP 1 932 534 A1**
(43) Date of publication of application: **18.06.2008**
(21) Application number: 06811711.8
(22) Date of filing: 05.10.2006
(51) Int. Cl.: A61K 38/00, A61P 9/00, A61P 15/00, A61P 17/14, A61P 19/02, A61P 27/02, A61P 35/00, A61P 43/00, C12N 5/00, C12N 15/00

(54) **METHOD OF INHIBITING ANGIOGENESIS BY USING EPHRIN B2**

(30) Priority: 05.10.2005 JP 2005292406
(71) Applicant: Aqumen Biopharmaceuticals K.K., Fukuoka-city, Fukuoka 810-0001 (JP); KYUSHU UNIVERSITY, Higashi-ku Fukuoka-city Fukuoka 812-0053 (JP)
(72) Inventor: FUJISAWA, Kimihiko, Fukuoka 8120053 (JP); ISHIBASHI, Tatsuro, Fukuoka 8120053 (JP); KAGIMOTO, Tadahisa, Fukuoka 8100001 (JP); SASA, Yukio, Fukuoka 8120053 (JP)
(74) Representative: Bramley, Sarah Jane
(86) International application number: PCT/JP2006/320423
(87) International publication number: WO 2007/043629

(57) **Abstract**

It is intended to provide a method of inhibiting angiogenesis or neoangiogenesis and a composition therefor, and a method which is useful in treating a disease or a disorder relating to angiogenesis or neoangiogenesis and a composition therefor. The above-described compositions contain an effective amount of Ephrin B2 and the above-described methods involve the step of administering an effective amount of Ephrin B2. These compositions and methods are useful in treating a disease or a disorder relating to angiogenesis or neoangiogenesis. Moreover, these compositions and methods can inhibit neoangiogenesis from the retina.

## Description

### TECHNICAL FIELD

This invention relates to methods and compositions for suppressing angiogenesis or neovascularization, and more particularly to the use of ephrinB2 therein.

### BACKGROUND

Angiogenesis is a hallmark of diverse ocular pathological conditions such as age related macular degeneration, diabetic retinopathy and retinopathy of premature. Angiogenic cascade is triggered by a number of mediators and chemokines. For example, endothelial cell receptor tyrosine kinases (RTKs), which are associated with the multi-step angiogenesis processes, have been recognized as critical mediators of angiogenesis. The first generation of angiogenic cytokines, including the vascular endothelial cell growth factors (VEGFs), fit well into the concept of sprouting capillaries. More recently, the angiopoietins/Tie2 system has been identified as a vessel assembly and maturation-mediating ligand-receptor system. VEGFNEGF receptors and angiopoietins/Tie2 receptor families also classified as RTKs.

Eph receptors, the receptors for ephrins, comprise the largest family of tyrosine kinase receptors, consisting of eight EphA receptors and six EphB receptors. The Eph receptor tyrosine kinase family represents a new class of RTKs. While this family has been originally identified as neuronal pathfinding molecules, its role in angiogenesis remains unclear. Knock-out mice and adult ephrinB2-lacZ transgenic mice experiments have identified EphB receptors and ephrinB ligands as crucial regulators of vascular assembly, orchestrating arteriovenous differentiation and boundary formation (non-patent documents 2, 3, and 5). On the other hand, gene-targeting experiments have revealed that ephrinB2 is an early marker of arterial endothelial cells, and its receptor EphB4 reciprocally marks venous endothelial cells in the vertebrate embryo (non-patent documents 1, 2, 4, and 6). Moreover, endothelial cells in adults maintain their asymmetric arteriovenous expression pattern, suggesting that the ephrinB/EphB system plays a role in controlling vascular homeostasis and possesses the possibility to control pathological angiogenesis in adults (non-patent documents 3 and 5). It has also been reported that administration of antagonist (e.g. antibody) of Eph receptor mainly inhibits cancerous neovascularization while administration of agonist stimulates the neovascularization. Thus, it is desirable to determine the mode of action of the ephrins, as well as how these molecules can be used to manipulate the vascular system, particularly in pathological conditions.

Diabetic retinopathy is a major cause of blindness of people at ages 20-65 in the United States. Pathological conditions essential to the diabetic retinopathy are retinal microangiopathy and subsequent ischemia. It is well known that hypoxia in retinopathy leads to retinal neovascularization. However, in vascular channels newly formed by the retinal neovascularizasion, blood flow will not perfuse so that the retina becomes ischemic. This is because the vascular channels are immature and diapedetic. Moreover, these vascular channels will often penetrate a vitreous chamber. An ideal treatment of diabetic retinopathy is to obtain mature vascular channels that can suppress such a neogenesis of blood vessel whose direction of extension is wrong and perfuse the hypoxic retina, as well as saving from a hypoxia.

Age related macular degeneration is the leading cause of adult blindness in Europe and the United States, and is one of the three major causes in Japan. Nature of the pathological condition is the ingress of new blood vessels from a choroid into a macula, while hemorrhage from these abnormal new blood vessels, retinal detachment and the like result in blindness. Therefore, suppressing generation of these abnormal new blood vessels is an essential method of treatment for maintaining visual function. Extension of new blood vessels is known to be suppressed by covering the new blood vessels with pigment epithelia, but the mechanism thereof is not very well known.

### REFERENCES

The following documents are incorporated herein by reference.
Patent document 1: United States Patent Application Publication No. 2004/0136983
Non-patent document 1: Adams, R.H., et al. (2001). Cell 104(1):57-69.
Non-patent document 2: Adams, R.H., et al. (1999). Genes Dev 13(3):295-306.
Non-patent document 3: Gale, N. W., et al. (2001). Dev Biol 230(2):151-160.
Non-patent document 4: Gerety, S. S., et al. (1999). Mol Cell 4(3):403- 414.
Non-patent document 5: Shin, D., et al. (2001). Dev Biol 230(2):139-150.
Non-patent document 6: Wang, H.U., et al. (1998). Cell 93(5):741- 753.

### DISCLOSURE OF THE INVENTION

### PROBLEMS TO BE SOLVED BY THE INVENTION

It is an object of the present invention to provide methods, compositions and preventive and/or therapeutic agents for suppressing angiogenesis or neovascularization. It is an object of the present invention to provide methods, compositions and preventive and/or therapeutic agents useful in treatment of a disease or disorder related to angiogenesis or neovascularization.

### MEANS FOR SOLVING PROBLEMS

The present invention provides a method for inhibiting DNA synthesis in arterial endothelial cells (or venous endothelial cells), which comprises contacting the arterial endothelial cells (or venous endothelial cells) with an effective amount of an ephrinB2.

In one embodiment, the above-mentioned DNA synthesis is induced by vascular endothelial cell growth factor (VEGF), basic fibroblast growth factor (bFGF) or platelet derived growth factor (PDGF).

The present invention also provides a method for inhibiting p44/p42 MAP kinase activation in arterial endothelial cells (or venous endothelial cells), which comprises contacting the arterial endothelial cells (or venous endothelial cells) with an effective amount of an ephrinB2.

In one embodiment, the above-mentioned p44/p42 MAP kinase activation is induced by VEGF, bFGF or PDGF.

The present invention also provides a method for inhibiting tube formation from arterial endothelial cells (or venous endothelial cells), which comprises contacting the arterial endothelial cells (or venous endothelial cells) with an effective amount of an ephrinB2.

In one embodiment, the above-mentioned tube formation is induced by VEGF, bFGF or PDGF.

In one embodiment, the above-mentioned contacting is performed by administering the ephrinB2 to a mammal comprising the arterial endothelial cells (or venous endothelial cells).

The present invention also provides a method for suppressing neovascularization from a retina, comprising administering an effective amount of an ephrinB2 to an individual.

In one embodiment, the above-mentioned individual is one with a pathological angiogenesis or neovascularization outside of the retina or one with a possibility of generating the pathological angiogenesis or neovascularization outside of the retina.

The present invention also provides a method for treatment of a disease or disorder related to angiogenesis or neovascularization, which comprises administering an effective amount of an ephrinB2 to an individual requiring the treatment.

The present invention also provides a composition for inhibiting DNA synthesis in arterial endothelial cells (or venous endothelial cells), which comprises an effective amount of an ephrinB2.

The present invention also provides a composition for inhibiting p44/p42 MAP kinase activation in arterial endothelial cells (or venous endothelial cells), which comprises an effective amount of an ephrinB2.

The present invention also provides a composition for inhibiting tube formation from arterial endothelial cells (or venous endothelial cells), which comprises an effective amount of an ephrinB2.

The present invention also provides a composition for suppressing neovascularization from a retina, which comprises an effective amount of an ephrinB2 to an individual.

The present invention also provides a pharmaceutical composition for treatment of a disease or disorder related to angiogenesis or neovascularization, which comprises an effective amount of an ephrinB2.

The present invention also provides an agent for inhibiting DNA synthesis in arterial endothelial cells (or venous endothelial cells), which comprises an effective amount of an ephrinB2.

The present invention also provides an agent for inhibiting p44/p42 MAP kinase activation in arterial endothelial cells (or venous endothelial cells), which comprises an effective amount of an ephrinB2.

The present invention also provides an agent for inhibiting tube formation from arterial endothelial cells (or venous endothelial cells), which comprises an effective amount of an ephrinB2.

The present invention also provides an agent for suppressing retinal neovascularization, which comprises an effective amount of an ephrinB2.

The present invention also provides an agent for treatment of a disease or disorder related to angiogenesis or neovascularization, which comprises an effective amount of an ephrinB2.

In one embodiment of the above-mentioned methods, compositions, as well as agents for inhibiting, suppressing and prevention or treatment, the above-mentioned ephrinB2 comprises an extracellular domain but not a membrane-spanning domain and a cytoplasmic domain of a native ephrinB2.

In another embodiment of the above-mentioned methods, compositions, as well as agents for inhibiting, suppressing and prevention or treatment, the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia.

In yet another embodiment of the above-mentioned methods, compositions, as well as agents for inhibiting, suppressing and prevention or treatment, the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, and diabetic retinopathy.

### EFFECT OF THE INVENTION

According to the present invention, methods and compositions for suppressing angiogenesis or neovascularization are provided. The compositions and methods of the present invention can suppress pathological angiogenesis or neovascularization without suppressing physiological blood flow. Since the compositions and methods of the present invention do not affect normal blood vessels, they are useful in treatment of diseases or disorders related to angiogenesis or neovascularization.

### BRIEF DESCRIPTION OF THE DRAWINGS

Figure 1A is a graph showing the effects of ephrinB2 treatment and EphB4 treatment on thymidine uptake in VEGF, bFGF, or PDGF-BB-treated HAoECs.
Figure 1B is a graph showing the effects of ephrinB2 treatment and EphB4 treatment on thymidine uptake in VEGF, bFGF, or PDGF-BB-treated HUVECs.
Figure 1C is a photograph showing the forms of endothelial cells stimulated with VEGF at 7 days for control (untreated), and groups treated with either sephrinB2 or sEphB4 and treated with both sephrinB2 and sEphB4.
Figure 2A is an electrophoretic photograph showing the effects of ephrinB2 on ERK phosphorylation in VEGF or bFGF-stimulated HAoECs.
Figure 2B is an electrophoretic photograph showing the effects of ephrinB2 on VEGF receptor 2 (KDR) autophosphorylation in VEGF-stimulated HAoECs.
Figure 3 is a graph showing a result of quantitation assay of neovascularization in a mouse cornea to which bFGF and ephrinB2 are co-administered.
Figure 4 is a fluorescence micrograph showing the forms of flat-mounted fluorescein-dextran perfused retinas for control (A) and sephrinB-treated model (B).
Figure 5 is a graph showing area ratios of nonperfused regions in a control model (OIR) and sephrinB2-treated model (OIR+ephrinB2).
Figure 6 is a scanning electron micrograph showing the forms of retinal surface of P 17 neonatal mouse for control model (OIR) (A) and sephrinB2-treated model (OIR+ephrinB2) (B).
Figure 7 is a graph showing the effects of mouse ephrinB2 treatment and human ephrinB2 treatment on thymidine uptake in bFGF -treated HUVECs.
Figure 8 is a fluorescence photograph showing the states of funduses provided with 10 ng/mL human ephrinB2 treatment (A), PBS treatment (B), and 10 ng/mL mouse ephrinB2 treatment (C).

### BEST MODE FOR CARRYING OUT THE INVENTION

EphrinB2/EphB4 system plays an important role in vasculogenesis and angiogenesis. The present invention is based on our findings that ephrinB2 suppressed endothelial cell (EC) DNA synthesis and both VEGF- and FGF2-induced p44/p42 MAP Kinase activation. EphrinB2 also inhibited EC tube formation. Moreover, according to the corneal micropocket assay in mice and quantification of cornea neovascularization, ephrinB2 suppressed bFGF-induced corneal angiogenesis. Accordingly, targeting ephrinB2/EphB4 and its anti-angiogenic signaling pathway may be beneficial in the treatment of angiogenesis-dependent diseases.

Prior to describing the invention in further detail, the terms used in this application are defined as follows unless otherwise indicated.

### Definitions

The term "ephrinB2", unless otherwise specified, refers to a polypeptide that (1) shares substantial sequence similarity with a native ephrinB2 or extracellular domain thereof, preferably the native human ephrinB2; and (2) possesses a biological activity involving the native ephrinB2 or extracellular domain. The term "ephrinB2" includes the native ephrinB2 (preferably the native human ephrinB2) and extracellular domain thereof, as well as an analog and variant thereof possessing the biological activity of the above-mentioned native ephrinB2 or extracellular domain. It is to be noted that sequence of the ephrinB2 is publicly known in the art and a person skilled in the art would fully recognize the sequence and position of the extracellular domain, membrane-spanning domain and cytoplasmic domain.

A "native" molecule, such as a native ephrinB2, is a molecule that exists without human intervention. However, a native ephrinB2 may also be the extracellular domain of an ephrinB2 that exists without human intervention.

A "full-length" ephrinB2 is an ephrinB2 that contains both an extracellular and an intracellular domain. The full-length ephrinB2 may be native, or it may be an analog or variant of a native ephrinB2.

A polypeptide that shares "substantial sequence similarity" with a native molecule is at least about 30% identical with the native molecule at the amino acid level. The polypeptide is preferably at least about 40%, 50%, 60%, 70%, 80%, 85%, 90%, 95%, more preferably at least about 98%, further preferably about 99% and further more preferably 100% identical with the native molecule at the amino acid level.

The term "percent identity" or "% identity" of an analog or variant with a native molecule refers to the percentage of amino acid sequence in the native molecule which are also found in the analog or variant when the two sequences are aligned. Percent identity can be determined by any methods or algorithms established in the art, such as LALIGN, ClustalW or BLAST.

A polypeptide possesses a "biological activity" of a native ephrinB2 if it is capable of binding to the receptor for the native ephrinB2 or inhibiting EC DNA synthesis, extracellular-signal-regulated kinase (ERK) phosphorylation in EC, EC tube formation, angiogenesis or neovascularization. The activity to inhibit EC DNA synthesis, ERK phosphorylation in EC, EC tube formation, angiogenesis or neovascularization can be determined by any methods known in the art, particularly as described in the present application.

An "effective amount" is an amount of a substance sufficient to achieve the intended purpose. For example, an effective amount of an ephrinB2 to inhibit DNA synthesis is an amount sufficient, in vivo or in vitro, as the case may be, to result in a reduction in the amount of DNA synthesis. An effective amount of an ephrinB2 to treat a disease or disorder is an amount of the ephrinB2 sufficient to reduce or remove the symptoms of the disease or disorder. The effective amount of a given substance will vary with factors such as the nature of the substance, the route of administration, the size and species of the animal to receive the substance, and the purpose of giving the substance. The effective amount in each individual case may be determined empirically by a skilled artisan according to established methods in the art.

The term "individual" refers to an arbitrary animal, preferably human or non-human mammal, more preferably mouse, rat, other rodents, rabbit, dog, cat, pig, cow, horse or primate, further more preferably human.

A "pathological angiogenesis or neovascularization" may be formation or neogenesis of blood vessels that are not in a physiologically normal state. Such blood vessels, being immature and diapedetic, may become blood vessels where the blood may not be perfused. Also, the "pathological angiogenesis or neovascularization" may be an angiogenesis or neovascularization in a manner different from a physiologically normal state. For example, the term may refer to formation of new blood vessels by receiving stimulus such as inflammation.

An "individual with a pathological angiogenesis or neovascularization or with a possibility of generating the pathological angiogenesis or neovascularization" is an individual in which the above-mentioned angiogenesis or neovascularization has already been generated or an individual which may have a factor that would generate such an angiogenesis or neovascularization. The latter individual may be an individual who has been affected by a disease or disorder which may show symptoms of generating a "pathological angiogenesis or neovascularization" but has not yet shown such symptoms, an individual who has received stimulus which would generate a "pathological angiogenesis or neovascularization", and the like.

A "pathological angiogenesis or neovascularization" may be generated "intraretinally" or "extraretinally". "Extraretinal" refers to outside of retinal tissue. These types of angiogenesis or neovascularization include, for example, formation or neogenesis of blood vessels directed from a retina penetrated through an inner limiting membrane into a vitreous body and formation or neogenesis of blood vessels directed from a retina to a choroid.

The term "disease or disorder related to angiogenesis or neovascularization" relates to any disease or disorder which generates the above-mentioned "pathological angiogenesis or neovascularization", or causes an individual to malfunction due to "pathological angiogenesis or neovascularization". For such a disease or disorder, age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia can be mentioned but not limited thereto.

"Treating" a disease or disorder is to reduce or completely remove the symptoms of a disease or disorder (also called "curing"), or to prevent or delay the disease or disorder from developing.

The term "require treatment" refers to a decision made by a caregiver (e.g. doctor, nurse, clinical nurse, etc. for human; veterinarian for animal (including non-human mammal)). This decision is made based on various factors which are within the range of opinion of the caregiver, while including recognition that the individual is currently ill or may become ill in the future as a result of a certain state that is treatable with an arbitrary composition.

The term "composition" includes at least one active component, and relates to a combination of substances for producing effects of the active component in cells, tissues, organs or animals (preferably mammal, more preferably mouse, rat, other rodents, rabbit, dog, cat, pig, cow, horse or primate, further more preferably human) to which the composition is applied or administered. One skilled in the art will understand and recognize an appropriate technique for determining whether or not the active component has an effective result desired based on the requirement of the one skilled in the art.

A "pharmaceutical composition" includes at least one active component, and relates to a combination of substances for producing pharmaceutical effects of the active component in animals, preferably mammal, more preferably mouse, rat, other rodents, rabbit, dog, cat, pig, cow, horse or primate, further more preferably human. One skilled in the art will understand and recognize an appropriate technique for determining whether or not the active component has an effective result desired based on the requirement of the one skilled in the art.

The terms "agent for inhibiting", "agent for suppressing", and "agent for prevention and/or treatment" relate to agents prepared in a form suitable for application or administration for producing effects of the active component. These agents may produce effects of the active component in cells, tissues, organs or animals (preferably mammal, more preferably mouse, rat, other rodents, rabbit, dog, cat, pig, cow, horse or primate, further more preferably human) to which the composition is applied or administered. (Effect of ephrinB2 on endothelial cells)

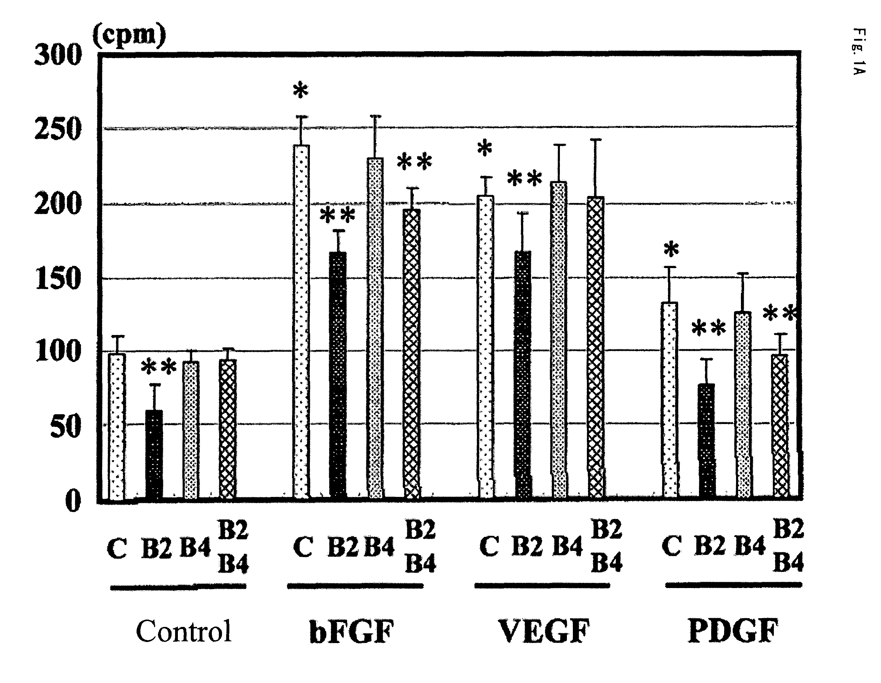

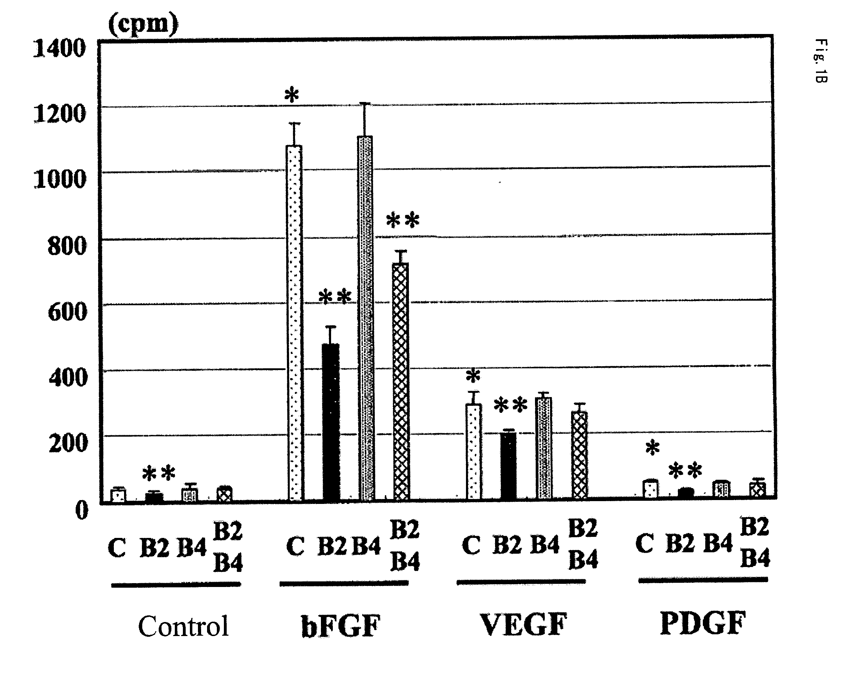

Endothelial cells can be induced to proliferate in response to growth factors, such as VEGF, bFGF, or PDGF-BB (B subunit dimer of PDGF). EphrinB2 inhibited DNA synthesis induced by all the stimuli of VEGF, bFGF, and PDGF-BB in arterial endothelial cells (or venous endothelial cells). As shown in Figure 1A, the DNA synthesis increase induced by 10 ng/mL of VEGF, bFGF, or PDGF-BB was inhibited by 200 ng/mL of ephrinB2, by 40%, 30%, and 90%, respectively. Virtually identical results were obtained using human umbilical vein endothelial cells (HUVECs) (Fig. 1B). This inhibitory effect of ephrinB2 on DNA synthesis was not caused by apoptosis.

Accordingly, ephrinB2 is capable of inhibiting EC DNA synthesis that is induced by various stimuli, including VEGF, bFGF and PDGF. While the receptor for ephrinB2 (EphB4) is a marker for venous endothelial cells, DNA synthesis was inhibited in both venous endothelial cells and arterial endothelial cells, although arterial endothelial cells are not known to possess this receptor.

In contrast, VEGF-induced tube formation was not affected by EphB4 treatment.

EphrinB2 inhibits the growth factor-induced mitogenic response. This is because ephrinB2 suppressed both VEGF and bFGF-induced ERK (p42/44) phosphorylation in both arterial and venous endothelial cells. However, ephrinB2 did not inhibit VEGF-receptor 2 autophosphorylation, indicating that ephrinB2 does not interfere with signal transduction between VEGF and VEGF-receptor 2 as a mechanism to inhibit VEGF functions. Thus, ephrinB2 can be used to inhibit VEGF or bFGF-induced ERK phosphorylation in either arterial or venous endothelial cells.

bFGF is known to be a potent angiogenic factor. EphrinB2 is capable of inhibiting EC cell proliferation induced by bFGF. Aadministration of ephrinB2 markedly blocked the angiogenesis induced by bFGF. In contrast, administration of EphB4 showed no effect.

### (Effect of ephrinB2 in in vivo model)

Corneal micropocket assay is a typical in vivo model of neovascularization. In this model, the angiogenesis induced by bFGF can be markedly blocked by administration of ephrinB2. In contrast, administration of EphB4 showed no effect.

Oxygen-induced retinopathy (OIR) model is an animal model of diabetic retinopathy. In this animal model, ephrinB2 is capable of suppressing pathological angiogenesis directed extraretinally, and intensifying formation of vascular network and vascular maturation within the retina.

Laser-induced choroidal neovascularization (CNV) model is positioned as an experimental disease-model for age-related macular degeneration (AMD). EphrinB2 is capable of suppressing the CNV.

Thus, ephrinB2 is capable of suppressing neogenesis of pathological angiogenesis directed extraretinally (e.g. choroidal neovascularization, inner limiting membrane spanning neovascularization) without suppressing physiological blood flow. Accordingly, ephrinB2 can be used to suppress pathological angiogenesis or neovascularization. Therefore, ephrinB2 is useful in the treatment of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia, as well as other diseases or disorders that are associated with angiogenesis or neovascularization.

In one embodiment, ephrinB2 is useful in the treatment of diseases or disorders that are associated with ocular angiogenesis or neovascularization. EphrinB2 does not cause damage to physiological blood flow of retina, which is usually caused by treatment with photodynamic therapy (PDT) and various antiangiogenic agents that are mainly used at present. Therefore, by using ephrinB2, wet and initial dry AMD may be treated, for example, by suppressing pathological choroidal neovascularization without causing damage to physiological blood flow of retina. Also, diabetic retinopathy may be treated by suppressing pathological inner limiting membrane spanning neovascularization without causing damage to physiological blood flow of retina.

### (Use and administration of ephrinB2)

The ephrinB2 that is useful in the present invention may be any ephrinB2, including analogs and variants, which possesses the required activity. Structure of the ephrinB2 is not limited as long as effect of the present invention is achieved. While the ephrinB2 contains the extracellular domain, it is possible that the ephrinB2 does not contain the membrane-spanning domain and the cytoplasmic domain, or the ephrinB2 may be full-length. Also, the ephrinB2 may be a shortened fragment.

The ephrinB2 used in the present invention may be obtained by isolating and purifying naturally-occurring protein or may be produced from microbes or the like by genetic recombination. For example, referring to the sequence of human ephrinB2 cDNA described in U.S. Patent No. 6,303,769 or Mol Immunol. 1995 Nov; 32(16):1197-205, full-length protein, protein including extracellular domain and the like may be prepared by using the conventional art. One skilled in the art may alter native ephrinB2 preferably by using a technique conventionally-used by the one skilled in the art. Also, any ephrinB2 commercially available as medical products or reagents may be used. Specifically, when using ephrinB2 for a pharmaceutical composition, ephrinB2 purified to a purity that is used for medical purposes is more preferable. For example, considering an immunogenic property, ephrinB2 may be altered into any form suitable for administration to an individual by fusing to Fc domain of immunogloblulin (preferably Fc domain of human IgG). It is to be noted that in examples that will be described later, protein with soluble ephrinB2 (usually contains an extracellular domain but not a membrane-spanning domain and a cytoplasmic domain) fused to human Fc is used due to its easy availability. However, this does not indicate that the effect of the present invention is limited to the ephrinB2 used in the examples.

Composition containing ephrinB2 may take various forms suitable for administering the composition to an individual (e.g. test animal) or for applying the composition to a sample (e.g. cell (e.g. endothelial cell, specifically arterial or venous endothelial cell), tissue, or organ). Methods for preparing such forms are well known in the art. Namely, the present invention also provides formulation suitable for predetermined usage of ephrinB2. While formulation suitable for predetermined usage of ephrinB2 is not specifically limited, " agent for inhibiting DNA synthesis in arterial endothelial cells (or venous endothelial cells)", "agent for inhibiting p44/p42 MAP kinase activation in arterial endothelial cells (or venous endothelial cells)", "agent for inhibiting tube formation from arterial endothelial cells (or venous endothelial cells)", "agent for suppressing retinal neovascularization", and the like can be mentioned. Such a formulation containing ephrinB2 may contain components other than ephrinB2 as long as effects of ephrinB2 are not blocked and the above-mentioned individual or sample is not given any harmful effect. Administration of such a formulation to an individual (e.g. test animal) or application of such a formulation to a sample (e.g. cell (e.g. endothelial cell, specifically arterial or venous endothelial cell), tissue, or organ) is performed in a manner that the ephrinB2 within the formulation contacts with the above-mentioned individual or sample.

Also, pharmaceutical composition containing ephrinB2 may be a formulation taking various forms suitable for administering the composition to an individual. Methods for preparing such forms are well known in the art. Namely, the present invention also provides formulation suitable for predetermined usage of ephrinB2. While formulation suitable for predetermined usage of ephrinB2 is not specifically limited, "agent for inhibiting DNA synthesis in arterial endothelial cells (or venous endothelial cells)", "agent for inhibiting p44/p42 MAP kinase activation in arterial endothelial cells (or venous endothelial cells)", "agent for inhibiting tube formation from arterial endothelial cells (or venous endothelial cells)", "agent for suppressing retinal neovascularization", "agent for prevention or treatment for treating a disease or disorder related to angiogenesis or neovascularization" and the like can be mentioned. While an expression "arterial endothelial cells (or venous endothelial cells)" is used in this specification, preferred embodiments of the present invention relate to arterial endothelial cells. The present invention also provides a pharmaceutical composition or an agent for prevention or treatment for treating a disease or disorder selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia. EphrinB2 is capable of treating or preventing at least one of the above-mentioned diseases or disorders. Such a composition containing ephrinB2 may contain an effective amount of ephrinB2 and a pharmaceutically acceptable carrier. This composition may contain other pharmaceutically acceptable components (including agents for suppressing neovascularization other than ephrinB2) as long as effects of ephrinB2 are not blocked. Pharmaceutical compositions or agents for prevention and/or treatment mainly containing ephrinB2 are also provided.

EphrinB2 can be administered systemically, e.g., orally or by intramuscular or intravenous injection, in admixture with a pharmaceutically acceptable carrier adapted for the route of administration. A variety of physiologically acceptable carriers can be used to administer ephrinB2 and their formulations are known to those skilled in the art and are described, for example, in Remington's Pharmaceutical Sciences (18th edition), ed. A. Gennaro, 1990, Mack Publishing Company, Easton, PA, and Pollock et al.

EphrinB2 is preferably administered parenterally (e.g., by intramuscular, intraperitoneal, intravenous, intraocular, intravitreal, or subcutaneous injection or implant). Formulations for parenteral administration include sterile aqueous or non-aqueous solutions, suspensions, or emulsions. A variety of aqueous carriers can be used, e.g., water, buffered water, saline, and the like. Examples of other suitable vehicles include polypropylene glycol, polyethylene glycol, vegetable oils, gelatin, hydrogenated naphalenes, and injectable organic esters, such as ethyl oleate. Such formulations may also contain auxiliary substances, such as preserving, wetting, buffering, emulsifying, and/or dispersing agents. Biocompatible, biodegradable lactide polymer, lactide/glycolide copolymer, or polyoxyethylene-polyoxypropylene copolymers may be used to control the release of the active ingredients.

Alternatively, ephrinB2 can be administered by oral ingestion. Formulations intended for oral use can be prepared in solid or liquid forms, according to any method known to the art for the manufacture of pharmaceutical compositions. The compositions may optionally contain sweetening, flavoring, coloring, perfuming, and preserving agents in order to provide a more palatable preparation.

Solid dosage forms for oral administration include capsules, tablets, pills, powders, and granules. Generally, these formulations contain active ingredient admixed with non-toxic pharmaceutically acceptable excipients.

These may include, for example, inert diluents, such as calcium carbonate, sodium carbonate, lactose, sucrose, glucose, mannitol, cellulose, starch, calcium phosphate, sodium phosphate, kaolin and the like. Binding agents, buffering agents, and/or lubricating agents (e.g., magnesium stearate) may also be used.

Tablets and pills can additionally be prepared with enteric coatings.

Liquid dosage forms for oral administration include pharmaceutically acceptable emulsions, solutions, suspensions, syrups, and soft gelatin capsules.

These forms contain inert diluents commonly used in the art, such as water or an oil medium, and can also include adjuvants, such as wetting agents, emulsifying agents, and suspending agents.

EphrinB2 can also be administered topically, for example, by patch or by direct application to the eye, or by iontophoresis.

EphrinB2 may be provided in sustained release compositions, such as those described in, for example, U.S. Patent Nos. 5,672,659 and 5,595,760. The use of immediate or sustained release compositions depends on the nature of the disorder being treated. If the disorder consists of an acute or over-acute disorder, treatment with an immediate release form will be preferred over a sustained release composition. Alternatively, for certain preventative or long-term treatments, a sustained released composition may be appropriate.

EphrinB2 may also be delivered using an implant. Such implants may be biodegradable and/or biocompatible implants, or may be non-biodegradable implants. The implants may be permeable or impermeable to the active ingredient. An ocular implant may be inserted into a chamber of the eye, such as the anterior or posterior chambers or may be implanted in the sclera, transchoroidal space, or an avascularized region exterior to the vitreous. In a preferred embodiment, the ocular implant may be positioned over an avascular region, such as on the sclera, so as to allow for transcleral diffusion of the drug to the desired site of treatment, e.g., the intraocular space and macula of the eye. Furthermore, the site of transcleral diffusion is preferably in proximity to the macula.

Examples of implants for delivery of ephrinB2 include, but are not limited to, the devices described in U.S. Patent Nos. 3,416,530; 3,828,777; 4,014,335; 4,300,557; 4,327,725; 4,853,224; 4,946,450; 4,997,652; 5,147,647; 5,164,188; 5,178,635; 5,300,114; 5,322,691; 5,403,901; 5,443,505; 5,466,466; 5,476,511; 5,516,522; 5,632,984; 5,679,666; 5,710,165; 5,725,493; 5,743,274; 5,766,242; 5,766,619; 5,770,592; 5,773,019; 5,824,072; 5,824,073; 5,830,173; 5,836,935; 5,869,079; 5,902,598; 5,904,144; 5,916,584; 6,001,386; 6,074,661; 6,110,485; 6,126,687; 6,146,366; 6,251,090; and 6,299,895, and in WO 01/30323 and WO 01/28474, all of which are incorporated herein by reference.

### (Dosage)

The amount of active ingredient that is combined with the carrier materials to produce a single dosage will vary depending upon the subject being treated and the particular mode of administration. Generally, ephrinB2 should be administered in an amount sufficient to reduce or eliminate a symptom of a disease.

Dosage levels on the order of about 1 µg/kg to 100 mg/kg of body weight per administration are generally useful in the treatment of neovascular disorders. When administered directly to the eye, the preferred administration is to result in intraocular concentration of about 1 ng/mL to about 100 ng/mL. The dosage may be administered as a single dose or divided into multiple doses. In general, the desired dosage should be administered at set intervals for a prolonged period, usually at least over several weeks, although longer periods of administration of several months or more may be needed.

One skilled in the art will appreciate that the exact individual dosages may be adjusted somewhat depending on a variety of factors: the time of administration; the route of administration; the nature of the formulation; the rate of excretion; the particular disorder being treated; the severity of the disorder; and the age, weight, health, and gender of the patient. Wide variations in the needed dosage are to be expected in view of the differing efficiencies of the various routes of administration. For instance, oral administration generally would be expected to require higher dosage levels than administration by intravenous or intravitreal injection. Variations in these dosage levels can be adjusted using standard empirical routines for optimization, which are well known in the art. The precise therapeutically effective dosage levels and patterns are preferably determined by the attending physician in consideration of the above-identified factors.

In addition to treating pre-existing neovascular diseases, ephrinB2 can be administered prophylactically in order to prevent or slow the onset of these disorders. In prophylactic applications, ephrinB2 is administered to a subject susceptible to or otherwise at risk of a particular neovascular disorder. Again, the precise amounts that are administered depend on various factors such as the individual's state of health, weight, etc.

While the present invention will be described below based on examples, the present invention is not limited to those examples.

### EXAMPLES

In the examples below, the following abbreviations have the following meanings. Abbreviations not defined have their generally accepted meanings.
°C= degree Celsius
hr = hour
min = minute
sec = second
µM = micromolar
mM = millimolar
M = molar
ml = milliliter
µl = microliter
mg = milligram
µg = microgram
DMEM = Dulbecco's modified Eagle's medium
ITS = Insulin-transferrin-selenium
FBS = fetal bovine serum
MEM = modified Eagle's medium
PBS = phosphate buffered saline
VEGF = vascular endothelial cell growth factor
FGF = fibroblast growth factor
PDGF = platelet derived growth factor
SDS = sodium dodecyl sulfate
PAGE = polyacrylamide gel electrophoresis
EC = endothelial cell
HUVEC = Human Umbilical Vein Endothelial Cell
HAoEC = Human Aortic Endothelial Cell
OIR = oxygen-induced retinopathy
CNV = choroidal neovascularization
AMD = age-related macular degeneration

In the examples, soluble proteins were used as ephrinB2 and EphB4 due to their easy availability (hereinafter, referred to as "sephrinB2" and "sEphB4", respectively; these are proteins that contain an extracellular domain but not a membrane-spanning domain and a cytoplasmic domain). Mouse sephrinB2 and sEphB4 were obtained from R&D Systems, Inc. (614 McKinley Place NE, Minneapolis 55413, USA) (both are in the form fused to human Fc domain). As described in example 5 below, human ephrinB2-human Fc synthesized protein is also used as ephrinB2.

The experiments shown below are repeated three times unless specifically mentioned, and data (means ± SD) from the representative experiment are indicated. Statistical significance was assumed when p < 0.05 using the Student t-test in normally distributed populations.

### (EXAMPLE 1: EphrinB2 Inhibits Proliferation and Migration of ECs Stimulated by Growth Factors)

To investigate effects if ephrinB2 and EphB4 on vascular endothelial cells stimulated by growth factors, [³H] thymidine uptake-DNA synthesis of HAoEC and HUVEC and endothelial tube assay were performed.

### (1. [³H] Thymidine uptake-DNA synthesis of HAoEC and HUVEC)

Human aortic epithelial cells (HAoECs) and human umbilical vein endothelial cells (HUVECs) were purchased from Clonetics Corp. (San Diego, California, USA) and maintained in Clonetics EGM medium supplemented with 10% fetal bovine serum (FBS). Endothelial cell growth supplements were also provided by Clonetics. HAoECs and HUVECs were cultured on type 1 collagen-coated dishes (Iwaki, Japan) in endothelial growth medium (Clonetics Corp., San Diego, California, USA) at 37°C in 5% CO2, 95% air, and the medium was changed every 2-3 days. Cells from passages 4 to 5 were used for experiments. ECs were treated for 18 hours in DMEM (Nacalai tesque, Japan) containing 10% FCS with 10 ng/mL of VEGF, bFGF, or PDGF-BB in the presence or absence of 200 ng/mL of either ephrinB2 or EphB4. The cells were then exposed to [methyl ³H] thymidine (Amersham) at 20 µCi/mL for 6 hours. The cells were trypsinized and retrieved onto glass fiber filters using an automatic cell harvester, and [methyl-³H] thymidine uptake was measured in a direct β counter. The results are shown in Figures 1A and 1B.

Treatment of human aortic epithelial cells (HAoECs) with VEGF, bFGF, or PDGF-BB increased DNA synthesis by 3-10 fold as compared with the untreated control. EphrinB2 inhibited DNA synthesis stimulated with all these stimulants, whereas neither sEphB4 nor sephrinB2+sEphB4 did. Figures 1A and 1B respectively show effects of sephrinB2 on DNA synthesis stimulated with VEGF, bFGF, or PDGF-BB for HAoECs (Figure 1A) and HUVEC (Figure 1B). Reference characters in the figures represent as follows: C: control (untreated), B2: sephrinB2-treated, B4: sEphB4-treated. In the figures, the mark * indicates that the result is significantly different from the untreated case without stimulation with growth factors, while the mark ** indicates that the result is significantly different from the case untreated with sephrinB2 or sEphB4 although stimulated with the same growth factors. As shown in Figure 1A, the DNA synthesis increase induced by 10 ng/mL of VEGF, bFGF, or PDGF-BB was inhibited by 200 µg/mL of sephrinB2, by 40%, 30%, and 90%, respectively. Virtually identical results were obtained using human umbilical vein endothelial cells (HUVECs) (Fig. 1B). No significant apoptotic cells were observed during this incubation period (data not shown).

### (2. Endothelial tube assay)

Collagen gels were formed by mixing together ice-cold gelation solution (10×M199, H₂O 0.53 M NaHCO₃, 200 mM L-glutamine, type I collagen, 0.1 M NaOH, 100:27.2:50:10:750:62.5 by volume) and cells in 1×basal medium (see below) at a concentration of 3×10⁶ cells/mL at a ratio of 4 volumes gelation solution:1 volume of cells. After gelation at 37 °C for 30 minutes, the gels were overlaid with 1×basal medium consisting of 1% FBS, 2 mM L-glutamine, 50 µg/ml ascorbic acid, 26.5 mM NaHCO₃, 100 units/ml penicillin, and 110 units/ml streptomycin, supplemented with 40 ng/ml bFGF, 40 ng/ml VEGF, and 80 nM PMA, 1×ITS. SephrinB2 and sEphB4 (200 ng/ml each) were added to the 1 × basal medium immediately after gelation. To quantitate tube formation, the number of tubes per high power (20 ×) field was determined 48 hours after addition of the basal medium. A tube was defined as an elongated structure comprised of one or more endothelial cells that exceeded 100 µm in length. Five independent fields separated by 100 µm optical sections were assessed for each well, and the average number of tubes/20×field was determined. Cytoxicity was assessed using a cell proliferation kit II from Boehringer Mannheim. Also, forms of VEGF-stimulated endothelial cells were observed at 7 days for control (untreated) and groups treated with either sephrinB2 or sEphB4 and treatment with both sephrinB2 and sEphB4

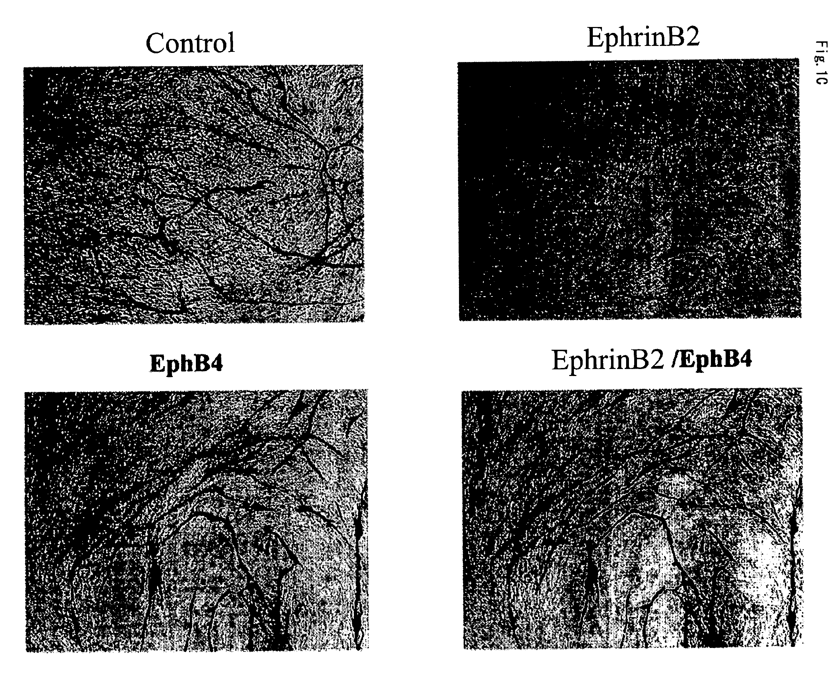

Figure 1C is a photograph showing the states of tube formation of endothelial cells stimulated with VEGF at 7 days for control (untreated), and groups treated with either sephrinB2 or sEphB4 and treated with both sephrinB2 and sEphB4. VEGF-induced tube formation was reduced in the sephrinB2 treated group compared with controls at 7 days (Figure 1C). In contrast, VEGF-induced tube formation was not affected by sEphB4 treatment.

Accordingly, ephrinB2 is capable of inhibiting EC DNA synthesis that is induced by various stimuli, including VEGF, bFGF and PDGF. Although the exact mechanism of ephrinB2 is not clear, the decrease in DNA synthesis was not caused by apoptosis, as no significant apoptosis was observed according to cytotoxic evaluation. It is surprising that DNA synthesis was inhibited in both venous endothelial cells and arterial endothelial cells, since the receptor for ephrinB2 (EphB4) is a marker for venous endothelial cells, while arterial endothelial cells are not known to possess this receptor.

Consistent with its effects on DNA synthesis, ephrinB2 is also capable of inhibiting EC tube formation that is induced by VEGF.

### (EXAMPLE 2: Effects of EphrinB2 on Growth Factor-Induced p42/44 ERK Phosphorylation and Receptor Autophosphorylation)

For the mechanism by which ephrinB2 inhibits the VEGF or bFGF-induced mitogenic response on endothelial cells, we investigated the effect of ephrinB2 on VEGF or bFGF-stimulated ERK (p42/44) phosphorylation in endothelial cells by Western analyses.

ECs were let stand for 1 hour in EGM-DMEM (containing 3% FCS) (Nacalai tesque, Japan) in the presence or absence of 50 ng/mL of sephrinB2. Further, the ECs were treated for 5 minutes with 10 ng/mL of bFGF or 10 ng/mL of VEGF (controls were left untreated). Preparation of protein samples from the endothelial cells and Western blotting were performed as follows: Whole cell lysates, cytosolic or nuclear extracts were isolated from endothelial cells. Western blotting was carried out both with and without immunoprecipitation. Protein samples were separated by sodium dodecyl sulfate-polyacrylamide gel electrophoresis (SDS-PAGE), followed by electrophoretic transfer to nitrocellulose membranes. After blocking with skim milk, the blots were incubated overnight at 4°C with antibodies (purchased from Santa Cruz Biotechnology (Santa Cruz, California, USA)) against phosphotyrosine or KDR (sc-504) (1:500). After washing, membranes were incubated with horseradish peroxidase-labeled second antibodies (Bio-Rad, Richmond, California, USA) (1:3000) for 1 hour at room temperature. Visualization was performed using Amersham enhanced chemiluminescence (ECL) detection system per the manufacturer's instructions.

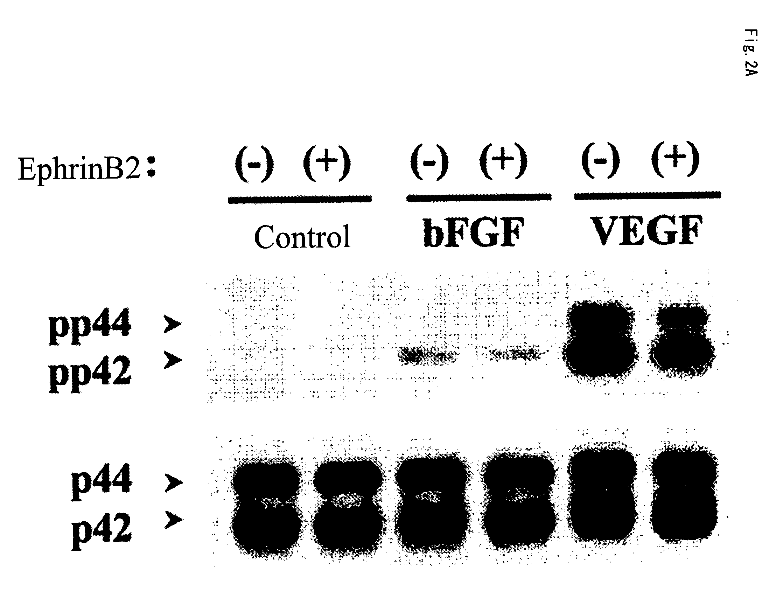

Figure 2A is an electrophoretic photograph showing the effects of ephrinB2 on ERK phosphorylation in VEGF or bFGF-stimulated HAoECs (p44/p42:total ERK. pp44/pp42: phosphorylated ERK). ERK phosphorylation increased by treatment with 10 ng/mL of VEGF or by treatment with 25 ng/mL of bFGF. SephrinB2 suppressed both VEGF and bFGF-induced ERK phosphorylation. For example, 200 µg/mL of sephrinB2 inhibited VEGF-induced ERK phosphorylation by 70%. Thus, it is seen that ephrinB2 inhibited VEGF and bFGF-stimulated ERK phosphorylation in HAoECs.

Next, we investigated VEGF-receptor 2 (KDR) autophosphorylation in VEGF-stimulated HUVECs. ECs were let stand for 1 hour in EGM-DMEM (containing 3% FCS) (Nacalai tesque, Japan) in the presence or absence of 50 ng/mL of sephrinB2. Further, the ECs were treated for 5 minutes with 10 ng/mL of bFGF or 10 ng/mL of VEGF (controls were left untreated). In the same way as above, the receptor (KDR) was immunoprecipitated (IP) from cell lysates, and blotting was carried out with phosphotyrosine antibody (PY20).

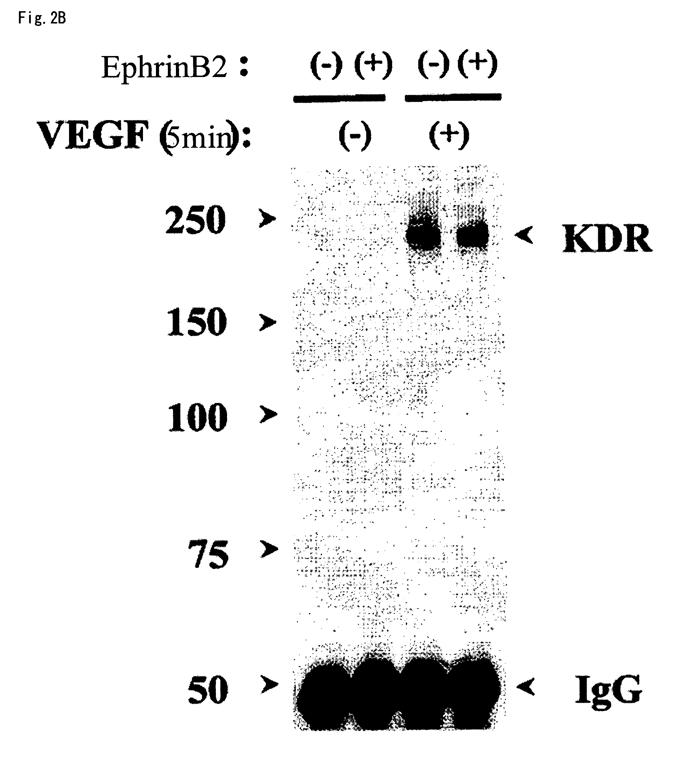

Figure 2B is an electrophoretic photograph showing the effects of ephrinB2 on VEGF receptor 2 (KDR) autophosphorylation in VEGF-stimulated HAoECs. Figure 2B shows that ephrinB2 has no remarkable effect on VEGF receptor 2 autophosphorylation in VEGF-stimulated HAoECs. VEGF-receptor 2 autophosphorylation was increased 14-fold by 10 ng/mL of VEGF. SephrinB2 did not inhibit VEGF-receptor 2 autophosphorylation.

Virtually identical results were obtained using HUVECs (data not shown) in the above-mentioned two phosphorylation assays.

These results thus indicate that ephrinB2 inhibits VEGF or bFGF-induced ERK phosphorylation in endothelial cells (arterial and venous). This effect probably accounts for, at least partially, the activity of ephrinB2 to inhibit VEGF or bFGF-induced proliferation of these cells. However, ephrinB2 does not inhibit autophosphorylation of VEGF-receptor 2. Therefore, ephrinB2 does not interfere with signal transduction between VEGF and VEGF-receptor 2 as a mechanism to inhibit VEGF functions.

### (EXAMPLE 3: Inhibition of bFGF-induced Angiogenesis by Co-administration of EphrinB2 in Mouse Corneas)

Basic FGF (bFGF) is known to be a potent angiogenic factor. Since ephrinB2 is capable of inhibiting EC cell proliferation induced by bFGF, we examined if ephrinB2 can suppress angiogenesis as well.

We essentially performed the corneal micropocket assay in mice and quantification of cornea neovascularization as previously described by Kenyon, B.M. et al., (1996) Ophthalmol. Vis. Sci., Vol. 37(8):1625-1632, with some modifications. Briefly, 0.3 µl of Hydron pellets (IFN Sciences, New Brunswick, New Jersey, USA) containing 90 ng of human bFGF were prepared and implanted in the corneas of male BALB/c mice. SephrinB2 or sEphB4 (100ng/pellet) was added directly to the bFGF/Hydron solution. The pellet was positioned 1.0 mm from the corneal limbus. After implantation, ofloxacin/eye drops were applied to each eye. After 6 days, the animals were sacrificed and the corneal vessels were photographed. The quantitative analysis of neovascularization in the mouse corneas was performed using the software package NTH Image. Six days after pellet implantation, we examined the outgrowth of new blood vessels. bFGF dramatically induced angiogenesis in mouse corneas.

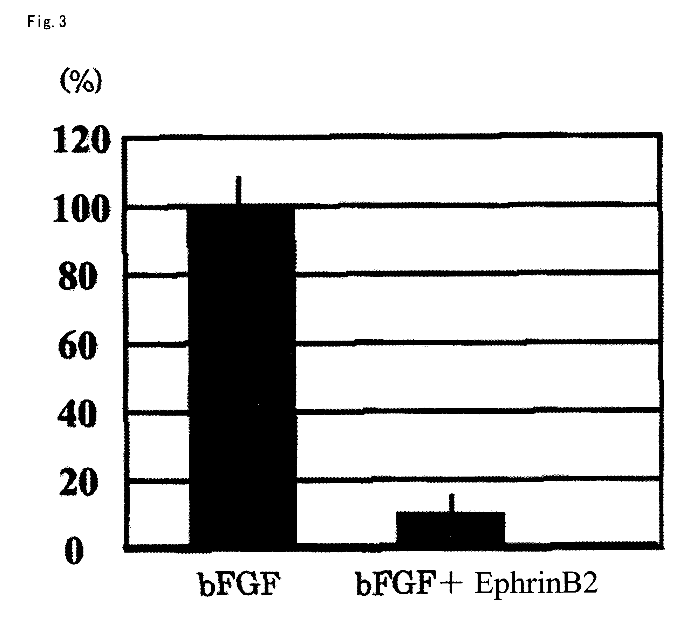

We further examined the effects of sephrinB2 and sEph B4 on bFGF-induced corneal neovascularization. Administration of sephrinB2 markedly blocked the angiogenesis induced by bFGF, however administration of EphB4 showed no effect. Figure 3 shows the result of quantitative analysis of angiogenesis in mouse corneas with co-administration of bFGF and ephrinB2. The longitudinal axis of Figure 3 indicates the value in terms of area of the region with neovascularization represented by percentage when the region with neovascularization in the presence of bFGF is represented by 100 %. Bars are SD for all animals in each group (n=6 per group). The quantitative analysis also demonstrated that bFGF-induced corneal neovascularization was completely inhibited by ephrinB2 (Figure 3).

### (EXAMPLE 4: Effects of EphrinB2 on Oxygen-induced Retinal Neovascularization)

Oxygen-induced retinopathy (OIR) model was prepared as follows by using C57BL/6J mice (obtained from SLC Japan). Mice at postnatal day 7 (P7) and their mother were put in a box which is made 75 % hyperoxic state for 5 days. 5 days after being put in the hyperoxic condition that is at postnatal day 12 (P12), the mice were returned to the normal condition of 20 % oxygen, and retinal vessel reaction thereafter was studied. The mice thus treated showed extensive progression of retinal neovascularization. The retinal neovascularization was formed in 100 % of the animals by P19. At P19, neovascular bundles were clear, and extended from the internal limiting membranes towards vitreous especially in the middle periphery.

To investigate effects of ephrinB2 on retinal neovascularization, 200 µg of sephrinB2 in 100 µL of PBS (n=6) and 200 µL of only PBS (n=6) as control were intraperitoneally injected once a day to OIR mice of P13 and P15. Retinal neovascularization was assessed at P17.

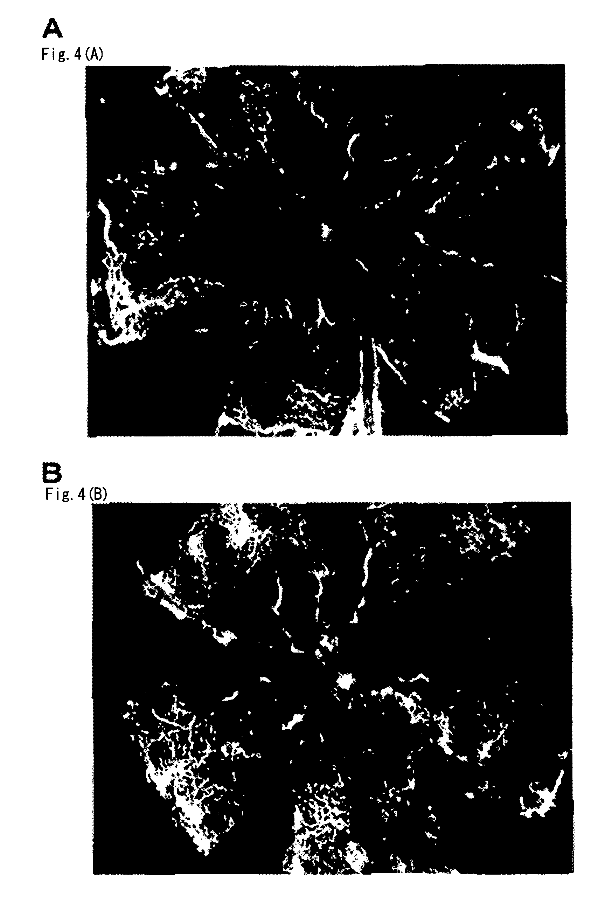

Following the protocol summarized below, flat-mounted retina was assessed using fluorescein-dextran angiography (Lab Invest. 2004; 84(8): 973-80). Firstly, mice were deeply anaesthetized and the left ventricle was perfused with 0.03 mL/g body weight of 50 mg/mL solution of 2×10⁶ molecular weight fluorescein-dextran (Sigma). Eyes were removed and fixed in 4 % paraformaldehyde for at least 3 hours. Then the corneas and lenses were removed, peripheral retinas were dissected and flat mounted on microscope slides for examination under a fluorescence microscope. A fluorescence micrograph of flat-mounted fluorescein-dextran perfused retinas for control and sephrinB-treated model is shown in Figure 4. (A: control OIR model; B: sephrinB-treated model). In the sephrinB-treated model, from the center to periphery of the retina is clearly white (Figure 4B). This indicates that normal blood vessels where the fluorescein-dextran can be perfused exist in the retina. Existence of the normal blood vessels was observed relatively more in the sephrinB-treated model compared to the OIR model (Figure 4A).

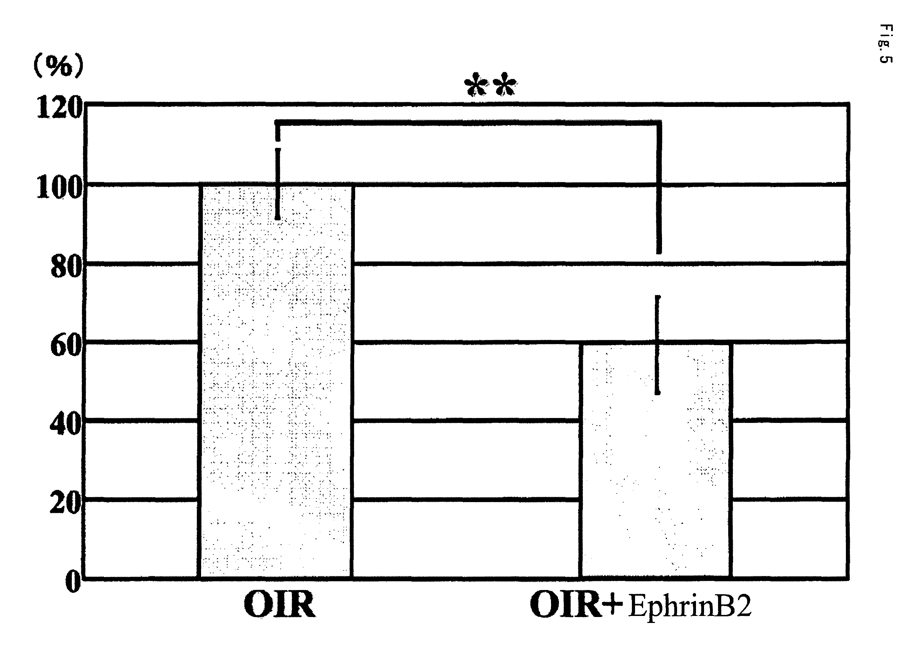

Also, the fluorescence micrograph of the flat-mounted fluorescein-dextran perfused retinas was taken into a software to measure the area of nonperfused regions. Figure 5 is a graph showing area ratios of nonperfused regions in a control model (OIR) and sephrinB2-treated model (OIR+ephrinB2). The longitudinal axis of Figure 5 indicates an area ratio of nonperfused regions, which is represented by percentage (%) when the nonperfused region in the OIR model is represented by 100 %. Bars are SD for all animals in each group (n=6 per group). It is seen from Figure 5 that sephrinB-treated model has reduced area of nonperfused region compared to the control model. Retinal avascularity ratio between the sephrinB2-treated model and the control model is statistically significant (noted with mark ** in Figure 5: p<0.01)

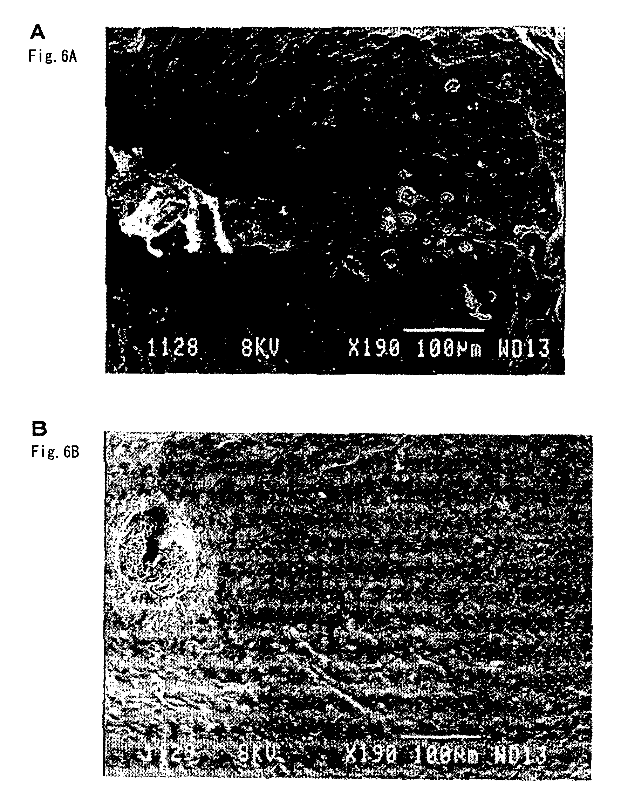

Moreover, effects of sephrinB2 on neogenesis of new blood vessels were examined with a scanning electron microscope. As previously described in Cell Tissue Res. (1992) 270:165-172, a scanning transmission electron microscopy was performed under an optimized condition for analyzing blood vascular system. Photographs were taken with Hitachi H-800 transmission microscope and S-800 scanning electron microscope. Figure 6 is a SEM photograph (retina was viewed from the side of vitreous body) showing the retinal surface of P17 neonatal mouse for control model (OIR) (A) and sephrinB2-treated model (OIR+ephrinB2) (B). In the photograph of the control model of Figure 6A, neogenesis of abnormal new blood vessels (inner limiting membrane spanning neovascularization) from the retina into the vitreous body was observed (specifically, the hole-like portion on the right side of the picture). Contrarily in the picture of the sephrinB2-treated model of Figure 6B, such a hole-like portion was not observed, so that neogenesis of new blood vessels into the vitreous body seems to have been suppressed.

Therefore, by treating the OIR mice with sephrinB2, the neogenesis of new blood vessels formed towards the vitreous body was suppressed although vascular network extending in virtually parallel direction to the membrane was observed. Also, the seprinB2 treatment did not decrease the blood flow itself of the retina. This is an important role of sephrinB2 in growth of blood vessels. SephrinB2 not only suppresses pathological neovascularization directed extraretinally (extending to the vitreous body in this example), but is capable of enhancing formation of vascular network and vascular maturation within the retina. Such an effect may be optimal for the treatment of diabetic retinopathy.

### (EXAMPLE 5: Suppressive effect of human EphrinB2 on Neovascularization)

In this example, fusion protein of ephrinB2 derived from human DNA library and human Fc, and neovascularization suppressive effect thereof was evaluated.

### (1. Preparation of human ephrinB2-human Fc synthetic protein)

As described below, a cDNA fragment of human ephrinB2 was fused to a 5' terminal of cDNA coding Fc portion of human IgG1 antibody. U.S. Patent No. 6,303,769 or Mol Immunol. 1995 Nov; 32(16):1197-205 was referred to for the sequence of this human ephrinB2 cDNA.

Fc cloning was performed as follows: PCR was carried outusing human spleen cDNA library (100 ng) as a template, forward primer: GAA GAT CTC CCA AAT CTT GTG ACA AAA CTC (sequence number 1) and reverse primer: GCG GCC GCT CAT TTA CCC GGA GA (sequence number 2),and KODplus (TOYOBO). The amplified DNA fragment of about 700 bp was purified, the purified DNA fragment was subcloned into Teasy vector (Promega), and confirmed to be human IgG1Fc.

Cloning of human ephrinB2 was performed as follows: PCR was carried out using human placental cDNA library (100 ng) as a template, forward primer: GCG AAG CTT ACC ATG GCT GTG AGA AGG GAC (sequence number 3) and reverse primer: GCG AGA TCT GGC CAC TTC GGA ACC GAG GAT (sequence number 4),and KODplus (TOYOBO). The amplified DNA fragment of about 680 bp was purified. When the purified DNA fragment was subcloned into Teasy vector (Promega), and base sequence was verified, it was 678 bp DNA fragment shown at 1-678 base positions in the base sequence of the above-mentioned human ephrinB2 cDNA. It is presumed that the protein coded by this human ephrinB2 DNA fragment includes the extracellular domain of human ephrinB2 protein but not the cytoplasmic domain and the membrane-spanning domain.

For the cloned EFN-B2 DNA fragment, 5' terminal was treated with HindIII, and 3' terminal was treated with Bg1II restriction enzyme, while for the Fc DNA fragment, 5' terminal was treated with Bg1II, and 3' terminal was treated with NotI restriction enzyme. For these fragments, three-piece ligation was carried out using mammalian expression vector pcDNA4-Myc-His A (Invitrogen) treated with HindIII and NotI restriction enzyme and DNA Ligation kit ver.2.1 (TAKARA). The vector obtained by the ligation was transformed into Escherichia coli XL21. When plasmid was purified from the grown Escherichia coli colony, and base sequence was verified, it was human EFN-B2(1-678bp)-Fc.

This plasmid was transfected into HEK293 cell and incubated under the following incubation conditions. As a medium, DMEM (SIGMA) supplemented with 10% FBS (Biowest Fetal Bovine Serum) and antibiotics (GIBCO 1% penicillin and 1% streptomycin) were used. Incubation was carried out in 5% CO₂ incubator for 3 days at 37°C. EphrinB2 stable cell line was obtained by gene transfection with calcium phosphate method and drug selection with Zeocin 250µg/mL (invivogen). When culture supernatant was collected from the stable cell line, incubation was carried out with GIT medium (Nihon Pharmaceutical Co., Ltd.).

Subsequently, expressed protein was purified as follows from the ephrinB2-Fc stable cell line.

About 100 mL of culture supernatant was collected and centrifuged (1100 rpm, 5 minutes, 4°C) to remove debris such as dead cells. 100 µL of protein A-sepharose was added thereto and rotated for 2 hours at 4°C). Thereafter, supernatant was removed by centrifugation (2000 rpm, 5 minutes, 4°C), and nonspecific absorbed protein and the like were removed by adding washing buffer (10 mM Tris-HCl pH7.4, 150 mM NaCl, 0.1% NP40) to precipitation (sepharose+ephrinB2). This washing operation was carried out 3 times. After washing for 3 times, 70µL of elution buffer (0.1M glycine pH3.0) was added, let stand on ice for 10 minutes, and precipitation was collected after centrifugation (2000 rpm, 2 minutes, 4°C). This operation was repeated 7 times. Protein concentration of the collected supernatant was measured at absorbance of OD₂₈₀. Those with high protein concentration was collected, neutralized by adding 1/10 quantity of 1M Tris-HCl pH8.0, and dialyzed overnight against phosphate-buffered saline. The sample was collected after dialysis, and quantitative determination of protein was carried out by BCA method. SDS-PAGE was carried out at the same time in a nonreduced or reduced state, and the sample was verified as human ephrinB2-human Fc synthetic protein by molecular weight.

### (2. [³H] Thymidine uptake-DNA synthesis of HUVEC)

In the same way as example 1, for the above-mentioned human ephrinB2-human Fc synthetic protein, we investigated effects of HUVEC on DNA synthesis. However, ECs were treated for 18 hours in DMEM (Nacalai tesque, Japan) containing 10% FCS with 0.6 nM of bFGF in the presence or absence of 1 nM, 10 nM, or 100 nM of human ephrinB2- Fc protein. As a positive control, 100 nM of mouse ephrinB2-human Fc chimeric protein (R&D Systems, Inc. (614 McKinley Place NE, Minneapolis 55413, USA)) was used.

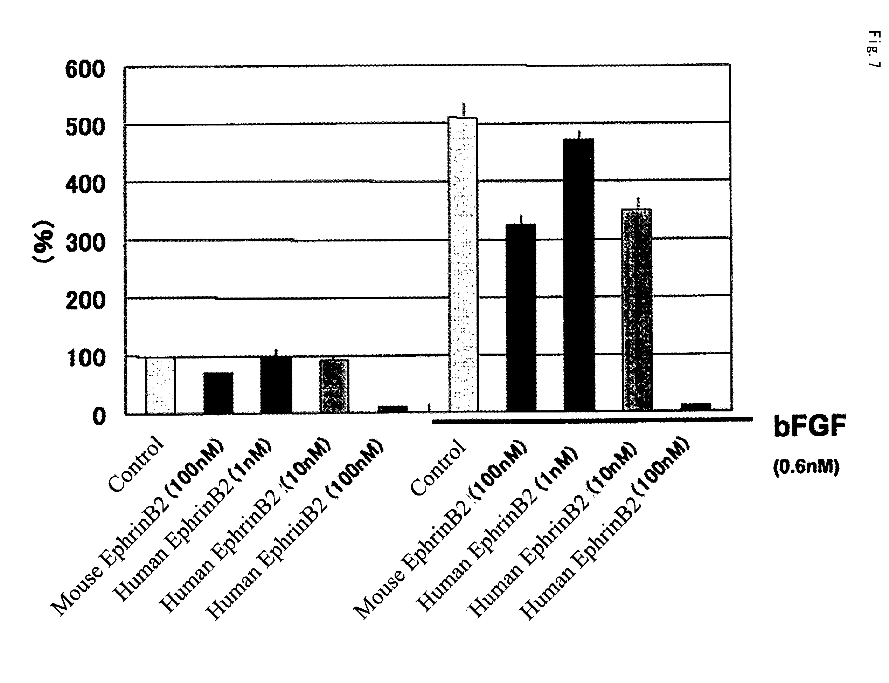

The result of thymidine uptake by HUVEC is shown in Figure 7. The longitudinal axis of Figure 7 indicates percentage (%) to the cases without treatment of bFGF and ephrinB2. When HUVEC was stimulated with bFGF (0.6 nM) without ephrinB2 treatment, DNA synthesis increased by 5-fold as compared with the bFGF-untreated control. 100 nM of human ephrinB2-human Fc synthetic protein inhibited DNA synthesis even without bFGF stimulation. Increase of DNA synthesis induced by 0.6 nM of bFGF was inhibited by 1 nM, 10 nM, and 100 nM of human ephrinB2- Fc synthetic protein by 10%, 30%, and 95%, respectively (Fig.7). No significant apoptotic cells were observed during this incubation period (data not shown).

### (EXAMPLE 6: Evaluation of EphrinB2 on CNV model)

Effects of human ephrinB2-human Fc synthetic protein on suppressing neovascularization was evaluated using laser-induced choroidal neovascularization (CNV) model that is considered as a biological model of AMD.

### (Experimental Animals)

12 eyes derived from 6 cynomolgus monkeys were used. These animals were all treated painlessly, and were picked up without pathogen from Shin Nippon Biomedical Laboratories, Ltd. Experiments were carried out under general anesthesia with ketamine hydrochloride. Experiments were carried out following the ethical standard (Approval No. c189-001) approved by Animal Experimentation Ethics Committee of Shin Nippon Biomedical Laboratories, Ltd.

### (Experimental Induction of Choroidal Neovascularization (CNV))

Experimental CNV was induced by photocoagulation using multicolor laser (wavelength 647 nm) (Novus Omni Laser; Lumenis, Santa Clara, Calif) in posterior pole of fundus of eyes of the above-mentioned monkeys. Size of the spot was 100 µm in diameter, and time of exposure was 0.1 sec. Output power at corneal surface was 700 mW. By using a contact lens, 8 burns were made in portions excluding the central fovia in each eye.

### (Administration Method and Concentration of EphrinB2)

Immediately after the laser irradiation and 5 days after the irradiation, namely twice in total, intravitreal administration of the human ephrinB2-human Fc synthetic protein prepared in the above-mentioned embodiment 5 was performed. 0.1 mL of PBS solution adjusted to make intravitreal ephrinB2 concentration respectively 1 ng/mL, 10 ng/mL, and 100 ng/mL was injected in the eye using 27G needle from the ciliary ring. As a negative control, 0.1 mL of PBS was injected. As a positive control, 10 ng/mL and 100 ng/mL of the above-mentioned mouse ephrinB2-human Fc chimeric protein were used.

### (Evaluation of CNV)

10 days after laser irradiation, fluorescein fundus angiography was carried out to evaluate choroidal neovascular. 0.1 mL/kg of 5% fluorecein Na was administered intravenously, and fluorescein fundus photography was carried out with a fundus camera (TRC-50EX, Topcon Corporation) Hiperlucency of choroidal neovascular was evaluated for the fluorescein fundus photographs 5-6 minutes after the start of injection. Evaluation on the photographs were carried out by an expert ophthalmology specialist. Except for 3 eyes which were impossible to evaluate due to opacity of optic media, lesion for every laser spot with significant hiperlucency of fluorochrome were determined to have activity.

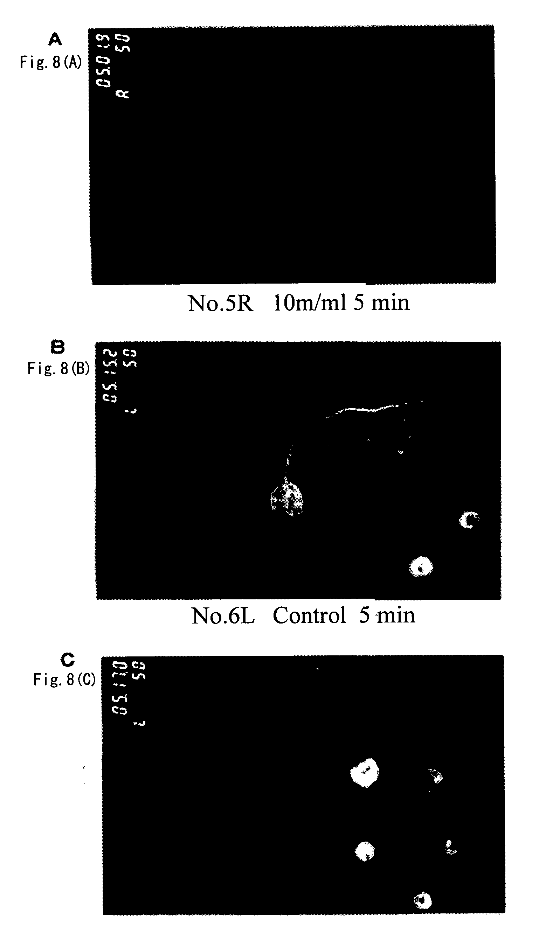

Figure 8 is a fluorescein photograph of funduses provided with the following treatments. Figure 8(A) is a result of 10 ng/mL human ephrinB2 treatment, Figure 8(B) is a result of negative control (PBS treatment), and Figure 8(C) is a result of 10 ng/mL mouse ephrinB2 treatment.

For the negative control with PBS injection, 10/16 were CNV with activity. On the other hand, in the ephrinB2 administered group the ratios of CNV active lesion were 5/16 for 1 ng/mL, 0/8 for 10ng/mL, and 3/8 for 100ng/mL. In the positive control group using 10 ng/mL and 100 ng/mL of mouse ephrinB2-human Fc chimeric protein, 5/8 and 5/16 were active CNV.

As described above, human ephrinB2-human Fc synthetic protein concentration-dependently suppressed proliferation in in vitro system using HUVEC. Specifically, with the concentration of 100 nM, the ability to suppress proliferation was 95%. Also, the human ephrinB2-human Fc synthetic protein suppressed generation of new blood vessels in laser-induced CNV mode. The intravitreous concentration of 10 ng/mL was effective.

EphrinB2 expressed on migrated pigment epithelia in the laser model (data not shown). Therefore, it is assumed that neovascularization was ceased by the expression of ephrinB2 on pigment epithelia. The result of this embodiment proves the effect of ephrinB2 on suppressing neovascularization.

### Industrial applicability

The compositions and methods of the present invention are useful in the treatment of diseases or disorders that are associated with angiogenesis or neovascularization. Specifically, the compositions and methods of the present invention are capable of extraretinal pathological angiogenesis and neovascularization without suppressing physiological blood flow within the retina. Therefore, the compositions and methods of the present invention can be used advantageously in the treatment of diseases or disorders that are associated with angiogenesis or neovascularization, such as AMD and diabetic retinopathy, compared to the current treatment method suggested by injection of PTD and other various anti-angeogenetic agents (e.g. anti-VEGF agent).

## Claims

1. A method for inhibiting DNA synthesis in arterial endothelial cells, comprising the step of contacting the arterial endothelial cells with an effective amount of an ephrinB2.

2. The method of claim 1, wherein the DNA synthesis is induced by an endothelial cell growth factor, a basic fibroblast growth factor or a platelet derived growth factor.

3. A method for inhibiting p44/p42 MAP kinase activation in arterial endothelial cells, comprising the step of contacting the arterial endothelial cells with an effective amount of an ephrinB2.

4. The method of claim 3, wherein the p44/p42 MAP kinase activation is induced by an endothelial cell growth factor, a basic fibroblast growth factor or a platelet derived growth factor.

5. A method for inhibiting tube formation from arterial endothelial cells, comprising the step of contacting the arterial endothelial cells with an effective amount of an ephrinB2.

6. The method of claim 5, wherein the tube formation is induced by an endothelial cell growth factor, a basic fibroblast growth factor or a platelet derived growth factor.

7. The method of any of claims 1-6 wherein the ephrinB2 comprises an extracellular domain but not a membrane-spanning domain and a cytoplasmic domain of a native ephrinB2.

8. The method of any of claims 1-7 wherein the ephrinB2 is administered to a mammal comprising the arterial endothelial cells.

9. A method for suppressing neovascularization from a retina, comprising administering an effective amount of an ephrinB2 to an individual.

10. The method of claim 9 wherein the individual is one with a pathological angiogenesis or neovascularization outside of the retina or one with a possibility of generating the pathological angiogenesis or neovascularization outside of the retina.

11. A method for treatment of a disease or disorder related to angiogenesis or neovascularization, comprising administering an effective amount of an ephrinB2 to an individual requiring the treatment.

12. The method of claim 11 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia.

13. The method of claim 11 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, and diabetic retinopathy.

14. The method of any of claims 9-13 wherein the ephrinB2 comprises an extracellular domain but not a membrane-spanning domain and a cytoplasmic domain of a native ephrinB2.

15. A composition for inhibiting DNA synthesis in arterial endothelial cells, comprising an effective amount of an ephrinB2.

16. A composition for inhibiting p44/p42 MAP kinase activation in arterial endothelial cells, comprising an effective amount of an ephrinB2.

17. A composition for inhibiting tube formation from arterial endothelial cells, comprising an effective amount of an ephrinB2.

18. A composition for suppressing neovascularization from a retina, comprising an effective amount of an ephrinB2.

19. A pharmaceutical composition for treatment of a disease or disorder related to angiogenesis or neovascularization, comprising an effective amount of an ephrinB2.

20. The composition of claim 19 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia.

21. The method of claim 19 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, and diabetic retinopathy.

22. The composition of any of claims 15-21 wherein the ephrinB2 comprises an extracellular domain but not a membrane-spanning domain and a cytoplasmic domain of a native ephrinB2.

23. An agent for inhibiting DNA synthesis in arterial endothelial cells, comprising an effective amount of an ephrinB2.

24. An agent for inhibiting p44/p42 MAP kinase activation in arterial endothelial cells, comprising an effective amount of an ephrinB2.

25. An agent for inhibiting tube formation from arterial endothelial cells, comprising an effective amount of an ephrinB2.

26. The agent of any of claims 23-25 wherein the ephrinB2 comprises an extracellular domain but not a membrane-spanning domain and a cytoplasmic domain of a native ephrinB2.

27. An agent for suppressing retinal neovascularization, comprising an effective amount of an ephrinB2.

28. The agent of claim 27 wherein the ephrinB2 comprises an extracellular domain but not a membrane-spanning domain and a cytoplasmic domain of a native ephrinB2.

29. An agent for prevention or treatment of a disease or disorder related to angiogenesis or neovascularization, comprising an effective amount of an ephrinB2.

30. The agent for prevention or treatment of claim 29 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, diabetic retinopathy, cancers, rheumatoid arthritis, endometriosis, and alopecia.

31. The agent for prevention or treatment of claim 29 wherein the disease or disorder is selected from a group consisting of age-related macular degeneration, ischemic retinopathy, intraocular neovascularization, corneal neovascularization, retinal neovascularization, choroidal neovascularization, diabetic macular edema, diabetic retina ischemia, diabetic retinal edema, and diabetic retinopathy.

32. The agent of any of claims 29-31 wherein the ephrinB2 comprises an extracellular domain but not a membrane-spanning domain and a cytoplasmic domain of a native ephrinB2.


SEQUENCE LISTING

<110> Aqumen Biopharmaceuticals K.K.
FuJISAWA, Kimihiko
ISHIBASHI, Tatsuro
KAGIMOTO, Tadahisa
SASA, Yukio

<120> Method of inhibiting angiogenesis by using Ephrin B2

<130> P44941EP-K

<140> EP 06811711.8
<141> 2006-10-05

<150> 2005-292406
<151> 2005-10-05

<160> 4

<170> PatentIn version 3.3

<210> 1
<211> 30
<212> DNA
<213> Artificial Sequence

<220>
<223> Primer

<400> 1
gaagatctcc caaatcttgt gacaaaactc 30

<210> 2
<211> 23
<212> DNA
<213> Artificial Sequence

<220>
<223> Primer

<400> 2
gcggccgctc atttacccgg aga 23

<210> 3
<211> 30
<212> DNA
<213> Artificial Sequence

<220>
<223> Primer

<400> 3
gcgaagctta ccatggctgt gagaagggac 30

<210> 4
<211> 30
<212> DNA
<213> Artificial Sequence

<220>
<223>  Primer

<400>  4
gcgagatctg gccacttcgg aaccgaggat                                        30